# EUROPÄISCHE PATENTSCHRIFT

(11) **EP 2 948 086 B1**
(45) Veröffentlichungstag und Bekanntmachung des Hinweises auf die Patenterteilung: **05.02.2020**
(21) Anmeldenummer: 14703785.7
(22) Anmeldetag: 20.01.2014
(51) Int. Cl.: A61B 18/14, A61B 18/16

(54) **BIPOLARES RESEKTOSKOP**
BIPOLAR RESECTOSCOPE
RÉSECTOSCOPE BIPOLAIRE

(30) Priorität: 24.01.2013 DE 102013001156
(43) Veröffentlichungstag der Anmeldung: 02.12.2015
(73) Patentinhaber: BOWA-electronic GmbH & Co. KG, 72810 Gomaringen (DE)
(72) Erfinder: KRÖBER, Thomas, 72810 Gomaringen (DE); DOPPELSTEIN, Alexander, 78549 Spaichingen (DE); HEINRICH, Martin, 72379 Hechingen (DE)
(74) Vertreter: Schneider, Peter Christian
(86) Internationale Anmeldenummer: PCT/EP2014/051048
(87) Internationale Veröffentlichungsnummer: WO 2014/114600

(56) Entgegenhaltungen:
- WO-A1-97/24074
- WO-A1-97/24994
- WO-A1-2013/006633
- US-A1- 2011 282 341
- US-B1- 6 632 193

## Beschreibung

### Gebiet der Erfindung

Die Erfindung betrifft ein bipolares Resektoskop, umfassend
- einen Außenschaft, der komplett aus einem elektrisch leitenden Material ausgebildet ist und der an seinem distalen Ende eine Mehrzahl von Rückflussöffnungen aufweist,
- einen in dem Außenschaft anordenbaren Innenschaft aus einem elektrisch leitenden Material mit einem an seinem distalen Ende angeordneten Isoliereinsatz aus einem elektrisch nicht leitenden Material,
- wobei über die Rückflussöffnungen (39) des Außenschaftes (37) zwischen dem Außenschaft (37) und dem Innenschaft (2) ein Rückfluss von Spülflüssigkeit ermöglicht wird,
- einen in dem Innenschaft anordenbaren Elektrodentransporteur,
- eine in dem Elektrodentransporteur längsverschieblich anordenbare erste Elektrode, die an ihrem dem distalen Ende abgewandten proximalen Ende mit einem ersten Anschluss eines Hochfrequenzgenerators verbindbar ist und die an ihrem distalen Ende gabelförmig geteilt ist und parallel zur Längsachse zwei parallele Schlingenführungsrohre aufweist, zwischen denen eine halbkreisförmige Schneidschlinge aufgespannt ist, und
- eine zweite Elektrode, die an ihrem proximalen Ende mit einem zweiten Anschluss des Hochfrequenzgenerators verbindbar ist.

### Stand der Technik

Aus der EP 1 221 903 B1 ist ein urologisches Resektoskop mit einer ersten Elektrode bekannt, die auch als eine bipolare Elektrode ausgebildet sein kann. Solche Resektoskope bestehen aus einem in die Urethra einführbaren Endoskopschaft, in den ein Elektrodentransporteur mit einer bipolaren Elektrode und einer in einem Führungsrohr angeordneten Optik (Endoskop) eingesetzt werden kann. Dabei weist der Elektrodentransporteur einen entlang seines Führungsrohres längs verschieblichen Schiebekörper auf, der parallel zur Längsachse des Führungsrohres eine Aufnahmeöffnung zur Aufnahme eines dem Operateur zugewandten proximalen Endes der Elektrode aufweist. Das proximale Ende der Elektrode ist über eine Befestigungseinrichtung in dem Schiebekörper fixierbar.

An seinem distalen Ende weist das bekannte Resektoskop einen Isoliereinsatz aus einem elektrisch nicht leitenden Material auf.

Problematisch bei einem bipolaren Resektoskop ist, dass die aktive oder Schneidelektrode zur Erzielung einer hohen Schneidwirkung eine relativ geringe Schneidfläche und die passive oder Neutralelektrode zur Vermeidung einer eigenen Schneidwirkung relativ großflächig ausgebildet sein muss.

Aus der EP 1 163 886 B1 ist ein bipolares Resektoskop bekannt, dessen schlingenförmige Schneidelektrode als erste Elektrode oberhalb ihrer halbkreisförmigen Schneidschlinge einen dachförmigen Isolationsträger aufweist, der auf seiner der Schneidschlinge abgewandten Seite eine freiliegende Elektrodenfläche aufweist, die als passive oder Neutralelektrode mit dem distalen Ende einer zweiten Elektrode verbunden ist.

Nachteilig dabei ist, dass zum einen die Neutralelektrode zusammen mit der Schneidelektrode ausgetauscht werden muss und dass zum anderen der zusätzliche Isolationsträger mit der freiliegenden Elektrodenfläche unerwünscht das Blickfeld der Endoskopoptik einschränkt.

Aus der DE 10 258 730 A1 ist ein bipolares Resektoskop bekannt, das ebenfalls als aktive Elektrode eine schlingenförmige Schneidelektrode und als passive Elektrode eine bandförmige Elektrode aufweist, die der Schneidschlinge in distaler Richtung vorgelagert und radial oberhalb der Schneidschlinge angeordnet ist. Auch dieses bekannte Resektoskop weist die oben genannten Nachteile auf.

Weiterhin ist aus der EP 0 921 759 B1 ein bipolares Resektoskop bekannt, das ebenfalls als aktive Elektrode eine schlingenförmige Schneidelektrode aufweist, die über einen Elektrodentransporteur längsverschieblich in einem Innenschaft gelagert ist. Als passive Elektrode dient das distale Ende eines gegenüber dem Innenschaft in proximaler Richtung zurückgesetzten Außenschaftes. Der elektrisch leitende Außenschaft weist mit Ausnahme seines distalen Endes nach außen hin eine Isolierung auf.

Nachteilig dabei ist, dass zum einen der Abstand in Längsrichtung zwischen erster und zweiter Elektrode relativ groß sein muss und unerwünscht in proximale Richtung verlagert ist und dass es zum anderen bei defekter Isolierung des Außenschaftes zu unerwünschten Stromdichten und damit zu unerwünschten Verbrennungen kommen kann.

Weiterhin ist aus der DE 25 21 719 A1 ein bipolares Resektoskop bekannt, das ebenfalls als aktive Elektrode eine schlingenförmige Schneidelektrode aufweist, die über einen Elektrodentransporteur längsverschieblich in einem Endoskopschaft gelagert ist. Als passive Elektrode oder Neutralelektrode dient ein zwischen einem distalen Isoliereinsatz und dem distalen Ende eines Endoskopschaftes angeordnetes, elektrisch leitendes Rohrstück.

Nachteilig dabei ist, dass das elektrisch leitende Rohrstück vom distalen Ende des Endoskopschaftes durch einen zusätzlichen Isolierring elektrisch getrennt sein muss. Dieser Isolierring muss als zusätzliches Teil mit dem distalen Ende des Endoskopschaftes einerseits und mit dem dem distalen Ende des Endoskopschaftes zugewandten proximalen Ende des Rohrstückes andererseits relativ kostenintensiv verbunden werden. Weiterhin nachteilig ist, dass das elektrisch leitende Rohrstück sowohl in radialer Richtung nach außen als auch in radialer Richtung nach innen Strom ableitet, was in Verbindung mit dem umliegenden Gewebe und der Stellung des Endoskopschaftes zu unterschiedlichen ableitenden Stromflächen und damit zu unterschiedlichen und veränderlichen Stromdichten führt.

Aus der WO 97/24074 A1 ist ein bipolares Resektoskop mit einem Schaft bekannt, der einen Metallschaft mit einer isolierenden Außenschicht und mit einer isolierenden Innenschicht umfasst. Am distalen Ende des Metallschaftes ist ein für die transurethrale Elektrochirurgie üblicher Isoliereinsatz angeordnet. An dem dem Isoliereinsatz zugewandten Ende des Metallschaftes ist ein Bereich als zweite Elektrode (neutrale Elektrode) zwischen der isolierenden Außenschicht und der isolierenden Innenschicht angeordnet. Der Isoliereinsatz weist keine leitende Elektrodenfläche auf.

Nachteilig bei dem bekannten bipolaren Resektoskop ist, dass der Metallschaft mit einer isolierenden Außenschicht und mit einer isolierenden Innenschicht versehen sein muss. Dies ist zum einen aufwendig und kostenintensiv. Zum anderen können insbesondere Beschädigungen oder Kratzer in der Außenschicht zu unerwünschten Harnröhrenstrikturen führen. Weiterhin nachteilig ist, dass die Elektrodenfläche in einem relativ großen Abstand zum Isoliereinsatz angeordnet sein muss und unerwünscht weit zurück in der Harnröhre positioniert ist.

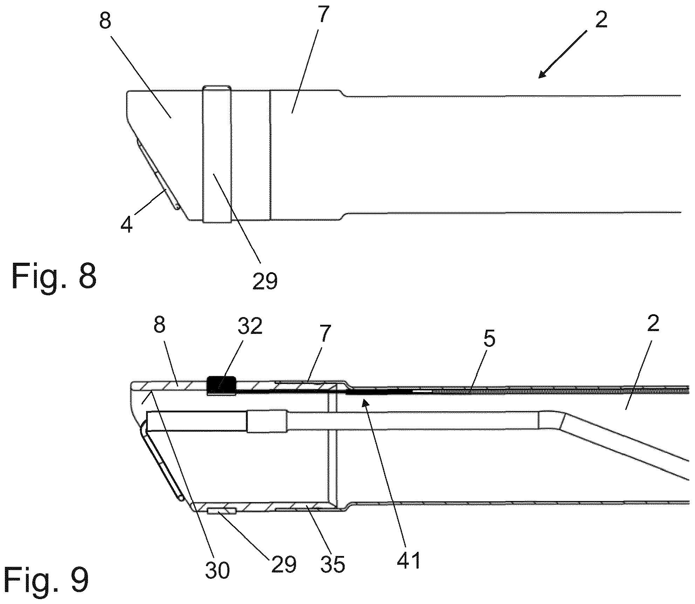

Aus der WO 97/24994 A1 ist ein elektrochirurgisches Instrument bekannt, das entsprechend der Ausführungsform E6 (s. Fig. 8 und 9) als eine bipolare Elektrode, eine "brush electrode", zur Arthroskopie ausgebildet ist. Ein isolierter Schaft weist an seinem distalen Ende eine neutrale Elektrode (return electrode) auf, die bereichsweise eine isolierte Keramikhülse umgibt, an der die aktive Elektrode in Form von Drähten (filaments) seitlich austritt. Derartige Elektroden sind nicht als Schneidelektroden in einem bipolaren Resektoskop verwendbar.

Aus der US 2011/0282341 A1 ist eine aktive Elektrodenanordnung zur Verbindung mit einer elektrochirurgischen Vorrichtung bekannt. Die bekannten Elektroden, die insbesondere zum Einsatz in der Arthroskopie vorgesehen sind, sind nicht geeignet, um als Schneidelektroden in Verbindung mit einem bipolaren Rektoskop eingesetzt zu werden.

Aus der WO 2013/006633 A1 ist eine bipolare elektrochirurgische Sonde zum Entnehmen von Gewebe bekannt. Diese bekannte Sonde wird in Verbindung mit einem Hysteroskop eingesetzt. Sie ist nicht geeignet in ein bipolares Resektoskop zur Transurethralen Resektion eingesetzt zu werden. Entsprechend weist sie auch keinen Isoliereinsatz eines Resektoskopschaftes auf.

### Aufgabenstellung

Aufgabe der vorliegenden Erfindung ist es daher, ein gattungsgemäßes biopolares Resektoskop derart weiterzubilden, dass die zweite Elektrode als passive Elektrode mit ausreichend großer Elektrodenfläche möglichst nah an der ersten Elektrode, die als aktive Elektrode ausgebildet ist, angeordnet ist. Wobei elektrische Sicherheit einerseits und eine möglichst geringe Beeinträchtigung des Sichtfeldes andererseits kostengünstig gegeben sein soll.

### Darstellung der Erfindung

Diese Aufgabe wird in Verbindung mit den Merkmalen des Anspruchs 1 dadurch gelöst, dass der Isoliereinsatz an seinem distalen Ende eine umlaufende, elektrisch leitende und quer zur Längsachse des Innenschaftes freiliegende Elektrodenfläche aufweist, die an der Innenseite des Isoliereinsatzes mit dem distalen Ende der zweiten Elektrode verbunden ist, dass die freiliegende Elektrodenfläche des Isoliereinsatzes in Längsrichtung einerseits zu dem distalen Ende des Isoliereinsatzes hin und andererseits zu dem freien Ende des Innenschaftes hin isoliert angeordnet ist, und dass die freiliegende Elektrodenfläche des Isoliereinsatzes an der der Längsachse radial abgewandten Außenseite des Isoliereinsatzes angeordnet und nach innen hin elektrisch isoliert ist.

Durch die Anordnung einer freiliegenden Elektrodenfläche am Isoliereinsatz des Innenschaftes entsteht eine passive Elektrodenfläche von ausreichender Größe in der Nähe der ersten Elektrode ohne die Sicht gegenüber der Verwendung eines üblichen komplett nicht leitenden Isoliereinsatzes einzuschränken. Für den Fachmann überraschend ergeben sich dabei sehr gute Schneideigenschaften im Gewebe. Der Außenschaft kann dabei komplett leitend oder auch komplett aus einem nicht leitenden Material ausgebildet sein. In beiden Fällen erfolgt keine Ableitung von Strom über den Außenschaft. Somit sind auch eine hohe elektrische Sicherheit und ein Schutz vor unerwünschten Verbrennungen durch Ableitung von Strom über den Außenschaft gegeben. Durch die Isolierung der freiliegenden Elektrodenfläche auf der Außenseite oder auf der Innenseite des Isoliereinsatzes gibt es jeweils eine definierte Elektrodenfläche. Überraschend zeigt sich hier, dass es trotz kurzer Stromwege zwischen den beiden Elektroden nicht zu einem die Schneidwirkung beeinträchtigenden Stromfluss kommt. Ein zusätzlicher, relativ kostenintensiver Isolierring gegenüber dem Innenschaft wird nicht benötigt.

Diese Aufgabe wird in Verbindung mit den Merkmalen des Anspruchs 2 weiterhin dadurch gelöst, dass der Isoliereinsatz an seinem distalen Ende eine umlaufende, elektrisch leitende und quer zur Längsachse des Innenschaftes freiliegende Elektrodenfläche aufweist, die an der Innenseite des Isoliereinsatzes mit dem distalen Ende der zweiten Elektrode verbunden ist, dass die freiliegende Elektrodenfläche des Isoliereinsatzes in Längsrichtung einerseits zu dem distalen Ende des Isoliereinsatzes hin und andererseits zu dem freien Ende des Innenschaftes hin isoliert angeordnet ist, und dass die freiliegende Elektrodenfläche des Isoliereinsatzes an der der Längsachse radial zugewandten Innenseite des Isoliereinsatzes angeordnet und nach außen hin elektrisch isoliert ist.

Gemäß einer bevorzugten Ausführungsform der Erfindung ist die erste Elektrode als eine aktive Schneidelektrode und die freiliegende Elektrodenfläche des Isoliereinsatzes als eine passive Neutralelektrode ausgebildet.

Gemäß einer weiteren Ausführungsform der Erfindung ist der Isoliereinsatz aus einem Kunststoff ausgebildet und die freiliegende Elektrodenfläche ist aus einem metallischen Werkstoff ausgebildet. Die Elektrodenfläche kann dabei in dem Isoliereinsatz eingebettet sein.

Alternativ kann der Isoliereinsatz aus einer nicht leitenden Keramik ausgebildet und die freiliegende Elektrodenfläche aus einer metallisierten und damit leitenden Keramik ausgebildet sein.

Gemäß einer weiteren bevorzugten Ausführungsform der Erfindung ist der Innenschaft in einem Außenschaft anordenbar und die beiden Schäfte bilden einen Dauerspülschaft mit kontinuierlicher Spülung.

Nach einer weiteren bevorzugten Ausführungsform der Erfindung ist das distale Ende des Außenschaftes gegenüber dem distalen Ende des Isoliereinsatzes in proximaler Richtung im Bereich des Isoliereinsatzes zurückgesetzt. Dabei weist der Außenschaft an seinem distalen Ende eine Mehrzahl von Rückflussöffnungen auf. Über die Rückflussöffnungen wird ein Rückfluss von Spülflüssigkeit zwischen dem Innenschaft und dem Außenschaft ermöglicht.

Nach einer weiteren bevorzugten Ausführungsform der Erfindung ist der Isoliereinsatz mit seinem proximalseitigen Ende in das distale Ende des Innenschaftes eingesteckt und das mit der Elektrodenfläche verbundene Zwischenstück ist über eine Steckverbindung mit dem distalen Ende der zweiten Elektrode verbunden.

Dadurch ist es möglich, den Isoliereinsatz mit der Elektrodenfläche leicht auszutauschen. Auch können unterschiedliche Isoliereinsätze mit unterschiedlichen Elektrodenflächen eingesetzt werden.

Weitere Merkmale und Vorteile der Erfindung ergeben sich aus der nachfolgenden speziellen Beschreibung und den Zeichnungen.

### Kurzbeschreibung der Zeichnungen

Es zeigen:
- Figur 1:: eine Seitenansicht eines bipolaren Resektoskopes mit gestrichelt angedeuteter Optik;
- Figur 2:: eine Seitenansicht des distalen Endes von Fig. 1 im Schnitt und vergrößerter Darstellung;
- Figur 3:: eine Seitenansicht eines bipolaren Resektoskopes einer zweiten Ausführungsform mit gestrichelt angedeuteter Optik;
- Figur 4:: eine Seitenansicht des distalen Endes von Fig. 3 im Schnitt und vergrößerter Darstellung;
- Figur 5:: eine Seitenansicht eines bipolaren Resektoskopes einer dritten Ausführungsform mit gestrichelt angedeuteter Optik;
- Figur 6:: eine Seitenansicht des distalen Endes von Fig. 5 im Schnitt und vergrößerter Darstellung;
- Figur 7:: eine Seitenansicht im Schnitt des bipolaren Resektoskopes von Fig. 5 ohne Optik;
- Figur 8:: eine Seitenansicht des distalen Endes eines Innenschaftes mit Isoliereinsatz in vergrößerter Darstellung;
- Figur 9:: eine Seitenansicht des distalen Endes von Fig. 8 im Schnitt;
- Figur 10:: eine Seitenansicht des distalen Endes von Fig. 8 mit herausgezogenem Isoliereinsatz und
- Figur 11:: eine Seitenansicht des distalen Endes von Fig. 10 im Schnitt.

### Beschreibung bevorzugter Ausführungsformen

Ein bipolares Resektoskop 1 besteht im Wesentlichen aus einem Innenschaft 2, einem Elektrodentransporteur 3, einer ersten Elektrode 4, einer zweiten Elektrode 5 und einer Optik 6.

Der Innenschaft 2 weist an seinem dem Patienten zugewandten distalen Ende 7 einen Isoliereinsatz 8 auf. In den Innenschaft 2 ist von dessen dem distalen Ende 7 abgewandten proximalen Ende 9 her der Elektrodentransporteur 3 einsetzbar und mit dem Innenschaft 2 verrastbar.

Der Elektrodentransporteur 3 weist ein Führungsrohr 10 auf, an dessen dem Operateur zugewandten proximalen Ende 11 ein Anschlussstück 12 zum Anschluss der Optik 6, die in dem Führungsrohr 10 geführt wird, angeordnet ist. Der Elektrodentransporteur 3 weist einen Fingergriff 13 und einen in distaler Richtung vorlagerten Anschlusskonus 14 auf, den der Elektrodentransporteur 3 mit einem das proximale Ende des Innenschaftes 2 bildenden Hauptkörper 15 verriegelbar ist.

Auf dem Führungsrohr 10 ist ein längsverschieblicher Schiebekörper 16 gelagert. Über ein federndes Gelenk 17 ist der Schiebekörper 16 mit dem Anschlussstück 12 verbunden und kann über einen Daumenring 18 gegen die Federkraft des Gelenkes 17 in Richtung Fingergriff 13 gedrückt werden. Der Schiebekörper 16 weist eine Aufnahmeöffnung zur Aufnahme eines proximalen Endes 19 der ersten Elektrode 4 auf. Der Schiebekörper 16 weist einen Führungskanal 20 auf, mit der Schiebekörper 16 auf dem Führungsrohr 10 geführt wird, wobei die Längsachse 21 des Führungsrohres 10 mit der Längsachse des Führungskanals 20 zusammenfällt. Quer zur Längsachse 21 weist der Schiebekörper 16 eine Steckeraufnahme 22 für einen nicht dargestellten Stecker, der an einem instrumentenseitigen Ende eines Hochfrequenzkabels mit einem ersten Anschluss eines ebenfalls nicht dargestellten Hochfrequenzgenerators verbindbar ist. Entsprechend ist die zweite Elektrode 5 an ihrem proximalen Ende mit einem zweiten Anschluss des Hochfrequenzgenerators verbindbar.

Die erste Elektrode 4 ist in an sich bekannter Weise an ihrem distalen Ende 23 gabelförmig geteilt und weist etwa parallel zur Längsachse 21 zwei parallele Schlingenführungsrohre 24 auf, zwischen denen eine halbkreisförmige Schneidschlinge 25 aufgespannt wird. In den Ausführungsbeispielen ist die Schneidschlinge 25 in einer proximalen Richtung zurückgebogen und bildet die gegen die Längsachse 21 bzw. gegenüber den Schlingenführungsrohren 24 einen spitzen Winkel 26.

Der Isoliereinsatz 8 weist an seinem distalen Ende 27 eine umlaufenden, elektrisch leitende und quer zur Längsachse 28 des Innenschaftes 2 freiliegenden Elektrodenfläche 29 auf, die an der Innenseite 30 des Isoliereinsatzes mit dem distalen Ende 31 der zweiten Elektrode 5 über ein Zwischenstück 32 verbunden ist.

Gemäß den Ausführungsbeispielen ist die erste Elektrode 4 als eine aktive Schneidelektrode ausgebildet, während die freiliegenden Elektrodenfläche 29 des Isoliereinsatzes 8 als eine passive Neutralelektrode zur Rückführung des Hochfrequenzstromes ausgebildet ist.

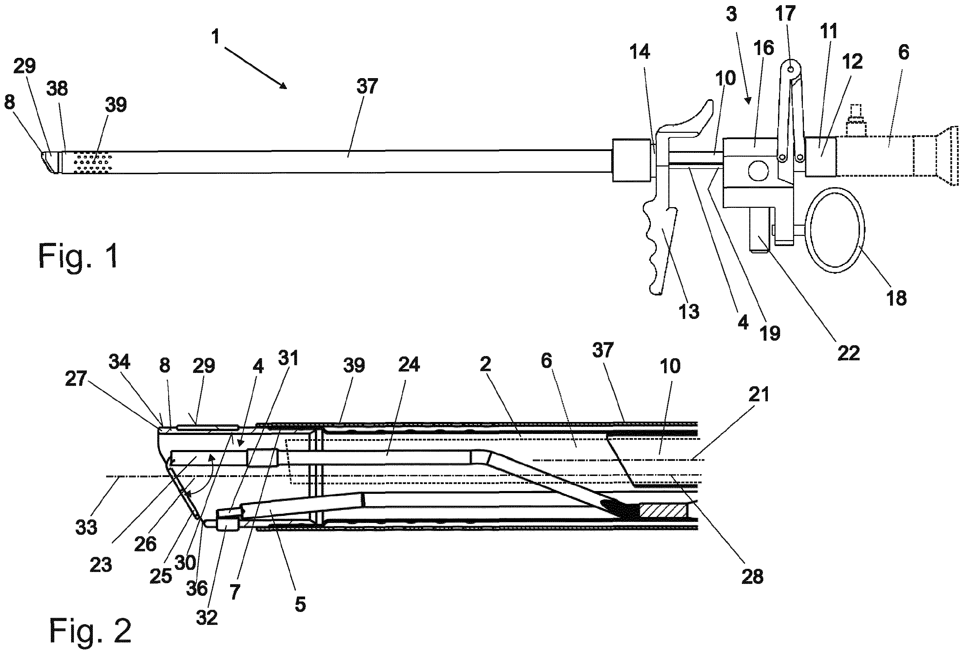

Gemäß den Ausführungsbeispielen der Figuren 1 bis 4 ist die freiliegende Elektrodenfläche 29 des Isoliereinsatzes 8 an der der Längsachse 33 radial abgewandten Außenseite 34 des Isoliereinsatzes 8 angeordnet, wobei die freiliegende Elektrodenfläche 29 des Isoliereinsatzes 8 nach innen hin, d.h. zur Innenseite 30 des Isoliereinsatzes 8 hin elektrisch isoliert ist.

Entsprechend den Ausführungsbeispielen der Figuren 1 und 2 ist die Elektrodenfläche 29 an der dachförmigen Oberseite des Isoliereinsatzes 8 breiter und im Bereich der Unterseite also im Bereich der Schneidkante 36 schmaler ausgebildet.

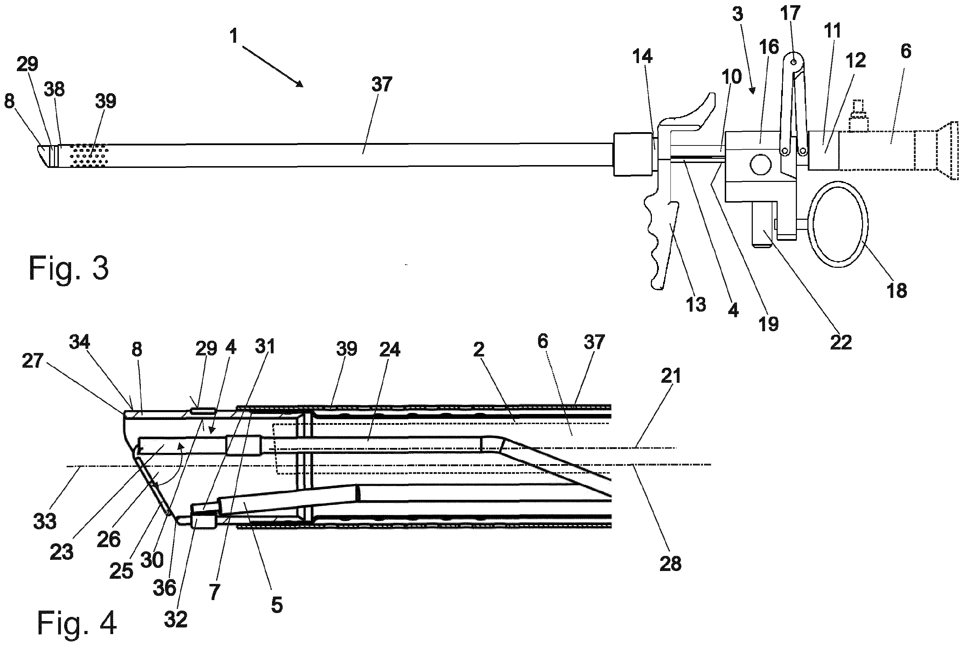

Entsprechend den Ausführungsbeispielen der Figuren 3 und 4 ist die freiliegende Elektrodenfläche 29 des Isoliereinsatzes 8 ringförmig ausgebildet. D.h., die Elektrodenfläche 29 ist sowohl im Dachbereich als auch im Bereich der Schneidkante 36 gleich breit ausgebildet.

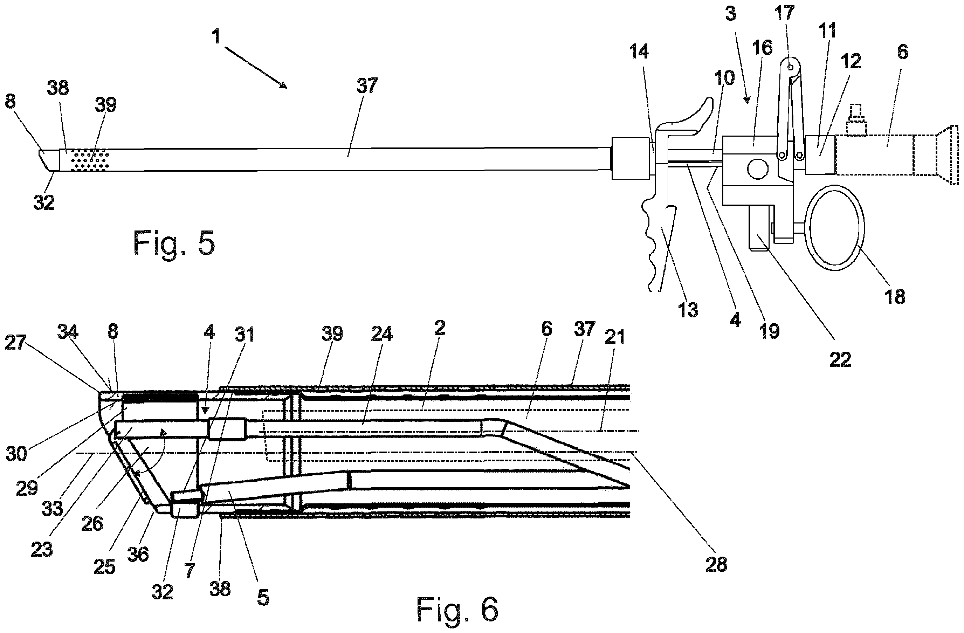

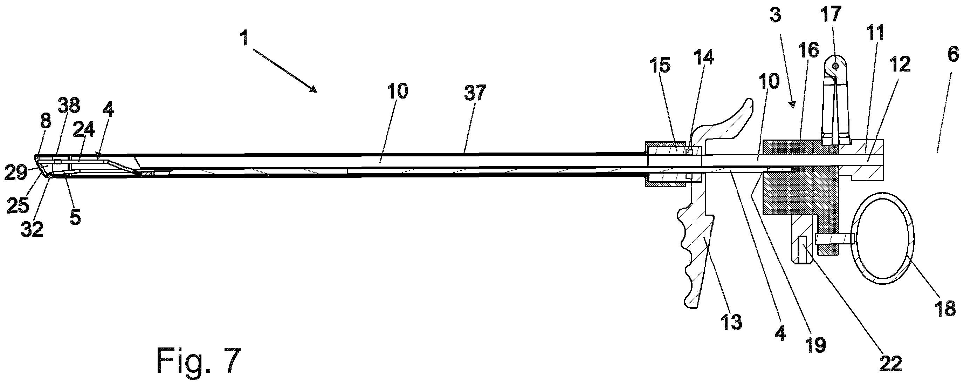

Entsprechend den Ausführungsbeispielen der Figuren 5 bis 7 ist die freiliegende Elektrodenfläche 29 des Isoliereinsatzes 8 an der der Längsachse 33 radial zugewandten Innenseite 30 des Isoliereinsatzes 8 angeordnet, wobei die die freiliegende Elektrodenfläche 29 des Isoliereinsatzes 8 radial nach außen hin elektrisch isoliert ist.

Die freiliegenden Elektrodenflächen 29 ist entsprechend den Ausführungsbeispielen in Längsrichtung einerseits zu dem distalen Ende 27 des Isoliereinsatzes 8 hin und andererseits zu dem freien oder distalen Ende 7 des Innenschaftes hin isoliert angeordnet.

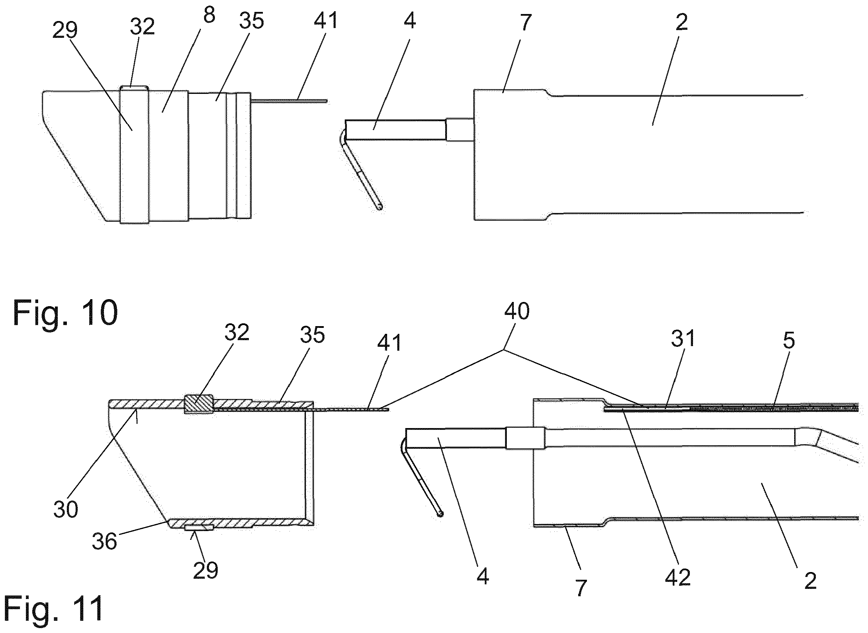

Entsprechend den Ausführungsbeispielen der Figuren 8 bis 11 ist der Isoliereinsatz (8) mit seinem proximalseitigen Ende 35 in das distale Ende 7 des Innenschaftes 2 eingesteckt. Das mit der Elektrodenfläche 29 verbundene Zwischenstück 32 ist über eine Steckverbindung 40 mit dem distalen Ende 31 der zweiten Elektrode 5 verbunden. Die Steckverbindung 40 besteht aus einem Steckkontakt 41, der aus dem proximalen Ende 35 des Isoliereinsatzes 8 herausragt und mit dem Zwischenstück 32 elektrisch leitend verbunden ist, und aus einer Kontakthülse 42, die das distale Ende 31 der zweiten Elektrode 5 bildet und die das freie Ende des Steckkontaktes 41 aufnimmt.

Der Isoliereinsatz 8 ist beispielsweise aus einem Kunststoff ausgebildet, wobei die freiliegende Elektrodenfläche 29 aus einem metallischen Werkstoff ausgebildet ist. Der Isoliereinsatz 8 kann aber auch aus einer nicht leitenden Keramik ausgebildet sein, wobei die freiliegende Elektrodenfläche 29 aus einer metallisierten Keramik ausgebildet ist.

Entsprechend den Ausführungsbeispielen ist der Innenschaft 2 in einem Außenschaft 37 angeordnet, wobei die beiden Schäfte 2, 37 einen Dauerspülschaft mit kontinuierlicher Spülung bilden. Dabei ist das distale Ende 38 des Außenschaftes 37 gegenüber dem distalen Ende 27 des Isoliereinsatzes 8 in proximaler Richtung im Bereich des Isoliereinsatzes 8 zurückgesetzt, also beabstandet.

Der Außenschaft 37 weist an seinem distalen Ende 38 eine Mehrzahl von Rückflussöffnungen 39 auf.

Entsprechend den Ausführungsbeispielen sind sowohl der Innenschaft 2 als auch der Außenschaft 37 aus einem elektrisch leitenden Material ausgebildet.

Natürlich stellen die in der speziellen Beschreibung diskutierten und in den Figuren gezeigten Ausführungsformen nur illustrative Ausführungsbeispiele der vorliegenden Erfindung dar. Dem Fachmann ist im Lichte der hiesigen Offenbarung ein breites Spektrum von Variationsmöglichkeiten an die Hand gegeben. Insbesondere kann der im Ausführungsbeispiel dargestellte passive Elektrodentransporteur auch als ein dem Fachmann bekannter aktiver Elektrodentransporteur ausgebildet sein. Selbstverständlich kann der Fachmann zum koagulieren von Gewebe die Schneidelektrode 4 durch eine dem Fachmann bekannte Koagulationselektrode ersetzen. Die in dem Innenschaft 2 angeordnete zweite Elektrode 5 muss nicht wie in den Ausführungsbeispielen gezeigt, unten im Bereich der Schneidkante 36 oder oben angeordnet sein. Der Fachmann wird sie im Hinblick auf die Anordnung von Optik und des nicht dargestellten Elektrodenführungsrohres des Elektrodentransporteurs 3 an geeigneter Stelle des Innenschaftes 2 anordnen.

### Bezugszeichenliste

- 1: bipolares Resektoskop
- 2: Innenschaft
- 3: Elektrodentransporteur
- 4: erste Elektrode
- 5: zweite Elektrode
- 6: Optik
- 7: distales Ende von 2
- 8: Isoliereinsatz von 2
- 9: proximales Ende von 2
- 10: Führungsrohr von 3
- 11: proximales Ende von 3
- 12: Anschlussstück von 3
- 13: Fingergriff von 3
- 14: Anschlusskörper von 3
- 15: Hauptkörper von 2
- 16: Schiebekörper von 3
- 17: Gelenk von 3
- 18: Daumenring von 16
- 19: proximales Ende von 4
- 20: Führungskanal von 16
- 21: Längsachse von 10
- 22: Steckeraufnahme
- 23: distales Ende von 4
- 24: Schlingenführungsrohr von 4
- 25: Schneidschlinge
- 26: Winkel von 25
- 27: distales Ende von 8
- 28: Längsachse von 2
- 29: Elektrodenfläche von 8
- 30: Innenseite von 8
- 31: distales Ende von 5
- 32: Zwischenstück von 5 und 8
- 33: Längsachse von 8
- 34: Außenseite von 8
- 35: proximales Ende von 8
- 36: Schneidkante von 8
- 37: Außenschaft
- 38: distales Ende von 37
- 39: Rückflussöffnungen
- 40: Steckverbindung
- 41: Steckkontakt von 8
- 42: Kontakthülse von 4

## Patentansprüche

1. Bipolares Resektoskop (1), umfassend
- einen Außenschaft (37), der komplett aus einem elektrisch leitenden Material ausgebildet ist und der an seinem distalen Ende eine Mehrzahl von Rückflussöffnungen (39) aufweist,
- einen in dem Außenschaft (37) angeordneten Innenschaft (2) aus einem elektrisch leitenden Material mit einem an seinem distalen Ende (7) angeordneten Isoliereinsatz (8) aus einem elektrisch nicht leitenden Material, wobei über die Rückflussöffnungen (39) des Außenschaftes (37) zwischen dem Außenschaft (37) und dem Innenschaft (2) ein Rückfluss von Spülflüssigkeit ermöglicht wird,
- einen in dem Innenschaft (2) anordenbaren Elektrodentransporteur (3),
- eine in dem Elektrodentransporteur (3) längsverschieblich anordenbare erste Elektrode (4), die an ihrem dem distalen Ende abgewandten proximalen Ende (19) mit einem ersten Anschluss eines Hochfrequenzgenerators verbindbar ist und die an ihrem distalen Ende (23) gabelförmig geteilt ist und parallel zur Längsachse (21) zwei parallele Schlingenführungsrohre (24) aufweist, zwischen denen eine halbkreisförmige Schneidschlinge (25) aufgespannt ist, und
- eine zweite Elektrode (5), die an ihrem proximalen Ende mit einem zweiten Anschluss des Hochfrequenzgenerators verbindbar ist,
wobei der Isoliereinsatz (8) an seinem distalen Ende (27) eine umlaufende, elektrisch leitende und quer zur Längsachse (28) des Innenschaftes (2) freiliegende Elektrodenfläche (29) aufweist, die an der Innenseite (30) des Isoliereinsatzes (8) mit dem distalen Ende (31) der zweiten Elektrode (5) verbunden ist,
wobei die freiliegende Elektrodenfläche (29) des Isoliereinsatzes (8) in Längsrichtung einerseits zu dem distalen Ende (27) des Isoliereinsatzes (8) hin und andererseits zu dem freien Ende (7) des Innenschaftes (2) hin isoliert angeordnet ist, und
wobei die freiliegende Elektrodenfläche (29) des Isoliereinsatzes (8) an der der Längsachse (33) radial abgewandten Außenseite (34) des Isoliereinsatzes (8) angeordnet und nach innen hin elektrisch isoliert ist.

2. Bipolares Resektoskop (1), umfassend
- einen Außenschaft (37), der komplett aus einem elektrisch leitenden Material ausgebildet ist und der an seinem distalen Ende eine Mehrzahl von Rückflussöffnungen (39) aufweist,
- einen in dem Außenschaft (37) anordenbaren Innenschaft (2) aus einem elektrisch leitenden Material mit einem an seinem distalen Ende (7) angeordneten Isoliereinsatz (8) aus einem elektrisch nicht leitenden Material, wobei über die Rückflussöffnungen (39) des Außenschaftes (37) zwischen dem Außenschaft (37) und dem Innenschaft (2) ein Rückfluss von Spülflüssigkeit ermöglicht wird,
- einen in dem Innenschaft (2) anordenbaren Elektrodentransporteur (3),
- eine in dem Elektrodentransporteur (3) längsverschieblich anordenbare erste Elektrode (4), die an ihrem dem distalen Ende abgewandten proximalen Ende (19) mit einem ersten Anschluss eines Hochfrequenzgenerators verbindbar ist und die an ihrem distalen Ende (23) gabelförmig geteilt ist und parallel zur Längsachse (21) zwei parallele Schlingenführungsrohre (24) aufweist, zwischen denen eine halbkreisförmige Schneidschlinge (25) aufgespannt ist, und
- eine zweite Elektrode (5), die an ihrem proximalen Ende mit einem zweiten Anschluss des Hochfrequenzgenerators verbindbar ist,
wobei der Isoliereinsatz (8) an seinem distalen Ende (27) eine umlaufende, elektrisch leitende und quer zur Längsachse (28) des Innenschaftes (2) freiliegende Elektrodenfläche (29) aufweist, die an der Innenseite (30) des Isoliereinsatzes (8) mit dem distalen Ende (31) der zweiten Elektrode (5) verbunden ist,
wobei die freiliegende Elektrodenfläche (29) des Isoliereinsatzes (8) in Längsrichtung einerseits zu dem distalen Ende (27) des Isoliereinsatzes (8) hin und andererseits zu dem freien Ende (7) des Innenschaftes (2) hin isoliert angeordnet ist, und
wobei die freiliegende Elektrodenfläche (29) des Isoliereinsatzes (8) an der der Längsachse (33) radial zugewandten Innenseite (30) des Isoliereinsatzes (8) angeordnet und nach außen hin elektrisch isoliert ist.

3. Bipolares Resektoskop nach Anspruch 1 oder 2,
**dadurch gekennzeichnet,**
**dass** die erste Elektrode (4) als eine aktive Schneidelektrode und die freiliegende Elektrodenfläche (29) des Isoliereinsatzes (8) als eine passive Neutralelektrode ausgebildet ist.

4. Bipolares Resektoskop nach einem der Ansprüche 1 bis 3,
**dadurch gekennzeichnet,**
**dass** der Isoliereinsatz (8) aus einem Kunststoff ausgebildet ist und
**dass** die freiliegende Elektrodenfläche (29) aus einem metallischen Werkstoff ausgebildet ist.

5. Bipolares Resektoskop nach einem der Ansprüche 1 bis 3,
**dadurch gekennzeichnet,**
**dass** der Isoliereinsatz (8) aus Keramik ausgebildet ist und
**dass** die freiliegende Elektrodenfläche (29) aus einer metallisierten Keramik ausgebildet ist.

6. Bipolares Resektoskop nach einem der Ansprüche 1 bis 5,
**dadurch gekennzeichnet,**
**dass** die beiden Schäfte (2, 37) einen Dauerspülschaft mit kontinuierlicher Spülung bilden.

7. Bipolares Resektoskop nach Anspruch 6,
**dadurch gekennzeichnet,**
**dass** das distale Ende (38) des Außenschaftes (37) gegenüber dem distalen Ende (27) des Isoliereinsatzes (8) in proximaler Richtung im Bereich des Isoliereinsatzes (8) zurückgesetzt ist.

8. Bipolares Resektoskop nach einem der Ansprüche 1 bis 7,
**dadurch gekennzeichnet,**
**dass** der Isoliereinsatz (8) mit seinem proximalseitigen Ende (35) in das distale Ende (7) des Innenschaftes (2) eingesteckt ist und
**dass** das mit der Elektrodenfläche (29) verbundene Zwischenstück (32) über eine Steckverbindung (40) mit dem distalen Ende (31) der zweiten Elektrode (5) verbunden ist.

## Claims

1. A bipolar resectoscope (1), comprising
- an outer shaft (37) made entirely of an electrically conductive material and having at its distal end a plurality of return flow openings (39),
- an inner shaft (2) arranged within the outer shaft (37), made of an electrically conductive material, with an insulating insert (8) made of an electrically nonconductive material arranged at its distal end (7), whereby a return flow of flushing fluid is enabled between the outer shaft (37) and the inner shaft (2) via the return flow openings (39) of the outer shaft (37),
- an electrode transporter (3) that can be arranged within the inner shaft (2),
- a first electrode (4) that can be arranged within the electrode transporter (3) in a longitudinally displaceable manner and that, at its proximal end (19) facing away from the distal end, can be connected to a first connection of a high frequency generator and is divided in a fork shape at its distal end (23) and has two parallel cutting loop guide tubes (24) parallel to the longitudinal axis (21), between which a semicircular cutting loop (25) is stretched open, and
- a second electrode (5) which can be connected at its proximal end to a second connection of the high-frequency generator,
wherein, at its distal end (27), the insulating insert (8) has a circumferential electrically conductive electrode surface (29) that is exposed transversely to the longitudinal axis (28) of the inner shaft (2), the exposed electrode surface being connected to the distal end (31) of the second electrode (5) on the inner side (30) of the insulating insert (8); wherein the exposed electrode surface (29) of the insulating insert (8) is arranged so as to be insulated in the longitudinal direction toward the distal end (27) of the insulating insert (8), on the one hand, and toward the free end (7) of the inner shaft, on the other hand; and
wherein the exposed electrode surface (29) of the insulating insert (8) is arranged on the outer side (34), facing radially away from the longitudinal axis (33), of the insulating insert (8), and is electrically insulated toward the inside.

2. A bipolar resectoscope (1), comprising
- an outer shaft (37) made entirely of an electrically conductive material and having at its distal end a plurality of return flow openings (39),
- an inner shaft (2) made of an electrically conductive material that can be arranged within the outer shaft (37), with an insulating insert (8) made of an electrically nonconductive material at its distal end (7), whereby a return flow of flushing fluid is enabled via the return flow openings (39) of the outer shaft (37) between the outer shaft (37) and the inner shaft (2),
- an electrode transporter (3) that can be arranged within the inner shaft (2),
- a first electrode (4) that can be arranged within the electrode transporter (3) in a longitudinally displaceable manner which, at its proximal end (19) facing away from the distal end, can be connected to a first connection of a high frequency generator and is divided in a fork shape at its distal end (23) and has two parallel cutting loop guide tubes (24) parallel to the longitudinal axis (21), between which a semi-circular cutting loop (25) is stretched open, and
- a second electrode (5), which can be connected at its proximal end to a second connection of the high-frequency generator,
wherein at its distal end (27), the insulating insert (8) has a circumferential electrically conductive electrode surface (29) that is exposed transversely to the longitudinal axis (28) of the inner shaft (2), the exposed electrode surface being connected to the distal end (31) of the second electrode (5) on the inner side (30) of the insulating insert (8); wherein the exposed electrode surface (29) of the insulating insert (8) is arranged so as to be insulated in the longitudinal direction toward the distal end (27) of the insulating insert (8), on the one hand, and toward the free end (7) of the inner shaft (2), on the other hand; and
wherein the exposed electrode surface (29) of the insulating insert (8) is arranged on the inner side (30), facing radially toward the longitudinal axis (33), of the insulating insert (8), and is electrically insulated toward the outside.

3. The bipolar resectoscope according to claim 1 or 2,
**characterized in that**
the first electrode (4) is designed as an active cutting electrode and the exposed electrode surface (29) of the insulating insert (8) is designed as a passive neutral electrode.

4. The bipolar resectoscope according to any of claims 1 to 3,
**characterized in that**
the insulating insert (8) is made of plastic and that the exposed electrode surface (29) is made of a metallic material.

5. The bipolar resectoscope according to any of claims 1 to 3,
**characterized in that**
the insulating insert (8) is made of a ceramic material and
the exposed electrode surface (29) is made of a metalized ceramic material.

6. The bipolar resectoscope according to any of claims 1 to 5,
**characterized in that**
the two shafts (2, 37) form a continuous flushing shaft with continuous flushing.

7. The bipolar resectoscope according to claim 6,
**characterized in that**
the distal end (38) of the outer shaft (37) is set back relative to the distal end (27) of the insulating insert (8) in the proximal direction, in the area of the insulating insert (8).

8. The bipolar resectoscope according to any of claims 1 to 7,
**characterized in that**
the proximal-side end (35) of the insulating insert (8) is inserted into the distal end (7) of
the inner shaft (2) and that
the adapter (32) connected to the electrode surface (29) is connected to the distal end (31) of the second electrode (5) via a plug connector (40).

## Revendications

1. Résectoscope bipolaire (1), comprenant
- une tige externe (37) constituée complètement d'un matériau électroconducteur et présentant une pluralité d'orifices de reflux (39) à son extrémité distale ,
- une tige intérieure (2) disposée dans la tige externe (37) et constituée d'un matériau électroconducteur, munie d'un insert isolant (8) agencé à son extrémité distale (7) et constitué d'un matériau non électroconducteur, un reflux du liquide d'irrigation étant rendu possible via les orifices de reflux (39) de la tige externe (37) entre la tige externe (37) et la tige intérieure (2),
- un transporteur d'électrode (3) pouvant être agencé dans la tige intérieure (2),
- une première électrode (4) pouvant être agencée pour coulisser longitudinalement dans le transporteur d'électrode (3), laquelle peut être connectée à son extrémité proximale (19) opposée à l'extrémité distale à un premier raccordement d'un générateur de haute fréquence et qui est divisée à son extrémité distale (23) en forme de fourche et présente deux tubes parallèles de guidage d'anse (24) parallèlement à l'axe longitudinal (21), entre lesquels est tendue une anse coupante (25) semi-circulaire, et
- une seconde électrode (5) pouvant être connectée à son extrémité proximale à un second raccordement au générateur de haute fréquence,
où l'insert isolant (8) présente à son extrémité distale (27) une surface d'électrode (29) périphérique électroconductrice qui est dégagée transversalement par rapport à l'axe longitudinal (28) de la tige intérieure (2) et qui est connectée sur le côté intérieur (30) de l'insert isolant (8) à l'extrémité distale (31) de la seconde électrode (5);
où la surface d'électrode (29) dégagée de l'insert isolant (8) est agencée, de manière isolée, dans la direction longitudinale, d'un côté, vers l'extrémité distale (27) de l'insert isolant (8) et, de l'autre côté, vers l'extrémité libre (7) de la tige intérieure (2);
et où la surface d'électrode (29) dégagée de l'insert isolant (8) est agencée sur le côté extérieur (34) de l'insert isolant (8), opposé radialement à l'axe longitudinal (33), et est isolée électriquement vers l'intérieur.

2. Résectoscope bipolaire (1), comprenant
- une tige externe (37) constituée complètement d'un matériau électroconducteur et présentant une pluralité d'orifices de reflux (39) à son extrémité distale,
- une tige intérieure (2) pouvant être disposée dans la tige externe (37) et constituée d'un matériau électroconducteur, munie d'un insert isolant (8) agencé à son extrémité distale (7) et constitué d'un matériau non électroconducteur, un reflux du liquide d'irrigation étant rendu possible via les orifices de reflux (39) de la tige externe (37) entre la tige externe (37) et la tige intérieure (2),
- un transporteur d'électrode (3) pouvant être agencé dans la tige intérieure (2),
- une première électrode (4) pouvant être agencée pour coulisser longitudinalement dans le transporteur d'électrode (3), laquelle peut être connectée à son extrémité proximale (19) opposée à l'extrémité distale à un premier raccordement d'un générateur de haute fréquence et qui est divisée à son extrémité distale (23) en forme de fourche et présente deux tubes parallèles de guidage d'anse (24) parallèlement à l'axe longitudinal (21), entre lesquels est tendue une anse coupante (25) semi-circulaire, et
- une seconde électrode (5) pouvant être connectée à son extrémité proximale à un second raccordement au générateur de haute fréquence,
où l'insert isolant (8) présente à son extrémité distale (27) une surface d'électrode (29) périphérique électroconductrice qui est dégagée transversalement par rapport à l'axe longitudinal (28) de la tige intérieure (2) et qui est connectée sur le côté intérieur (30) de l'insert isolant (8) à l'extrémité distale (31) de la seconde électrode (5);
où la surface d'électrode (29) dégagée de l'insert isolant (8) est disposée, de manière isolée, dans la direction longitudinale, d'un côté, vers l'extrémité distale (27) de l'insert isolant (8) et, de l'autre côté, vers l'extrémité libre (7) de la tige intérieure (2);
et où la surface d'électrode (29) dégagée de l'insert isolant (8) est agencée sur le côté intérieur (30) de l'insert isolant (8), tourné radialement vers l'axe longitudinal (33), et est isolée électriquement vers l'extérieur.

3. Résectoscope bipolaire selon la revendication 1 ou 2,
**caractérisé en ce que**
la première électrode (4) est réalisée comme une électrode de résection active et la surface d'électrode (29) dégagée de l'insert isolant (8) est réalisée comme une électrode neutre passive.

4. Résectoscope bipolaire selon l'une des revendications 1 à 3,
**caractérisé en ce que**
l'insert isolant (8) est constitué d'un matériau synthétique et que la surface d'électrode (29) dégagée est constituée d'un matériau métallique.

5. Résectoscope bipolaire selon l'une des revendications 1 à 3,
**caractérisé en ce que**
l'insert isolant (8) est en céramique et que
la surface d'électrode (29) dégagée est constituée d'une céramique métallisée.

6. Résectoscope bipolaire selon l'une des revendications 1 à 5,
**caractérisé en ce que**
les deux tiges (2, 37) forment une tige d'irrigation durable à irrigation continue.

7. Résectoscope bipolaire selon la revendication 6,
**caractérisé en ce que**
l'extrémité distale (38) de la tige externe (37) est en retrait par rapport à l'extrémité distale (27) de l'insert isolant (8) dans la direction proximale dans la zone de l'insert isolant (8).

8. Résectoscope bipolaire selon l'une des revendications 1 à 7,
**caractérisé en ce que**
l'insert isolant (8) est inséré par son extrémité proximale (35) dans l'extrémité distale (7) de la tige intérieure (2) et que
l'élément intercalé (32) raccordé à la surface d'électrode (29) est connecté à l'extrémité distale (31) de la seconde électrode (5) via une connexion enfichable (40).
